# FASCICULE DE BREVET EUROPEEN

(11) **EP 1 628 605 B1**
(45) Date de publication et mention de la délivrance du brevet: **04.07.2012**
(21) Numéro de dépôt: 04767212.6
(22) Date de dépôt: 28.05.2004
(51) Int. Cl.: A61F 9/007

(54) **INSTRUMENT DE CHIRURGIE OPHTALMIQUE**
INSTRUMENT FÜR DIE OPHTHALMISCHE CHIRURGIE
INSTRUMENT FOR OPHTHALMIC SURGERY

(30) Priorité: 28.05.2003 FR 0306465
(43) Date de publication de la demande: 01.03.2006
(73) Titulaire: Hehn, Frédéric, F-54180 Heillecourt (FR)
(72) Inventeur: Hehn, Frédéric, F-54180 Heillecourt (FR)
(74) Mandataire: Lewitter, Herbert
(86) Numéro de dépôt international: PCT/FR2004/001337
(87) Numéro de publication internationale: WO 2004/108033

(56) Documents cités:
- EP-A- 0 348 146
- EP-A- 0 657 150
- WO-A-99/65432
- DE-A- 19 904 995
- US-A- 4 578 059
- US-A- 5 674 226
- US-B1- 6 423 027

## Description

L'invention concerne un instrument de chirurgie ophtalmique, en particulier pour le nettoyage du sac capsulaire après extraction du cristallin atteint de cataracte.

La cataracte est caractérisée par une opacification du cristallin de l'oeil. Actuellement, le cristallin atteint de cataracte est extrait du sac capsulaire à travers une ouverture pratiquée dans la capsule antérieure et ensuite le cristallin est remplacé par un implant intraoculaire qui comporte une lentille pour restaurer la fonction réfractive du cristallin. Actuellement, l'extraction du cristallin est mise en oeuvre le plus souvent par un procédé dit de phaco-émulsification, suivi d'une aspiration des débris et un nettoyage du sac capsulaire.

Le sac capsulaire comporte une capsule antérieure antérieure et une capsule postérieure réunies à l'équateur. La face intérieure des capsules, ainsi que la zone équatoriale du sac capsulaire comportent une couche de cellules appelée épithélium, qui régénère le cristallin.

Une complication post-opératoire de cette intervention est la cataracte secondaire qui est due à la prolifération et la migration des cellules épithéliales résiduelles, avec pour conséquence une opacification de la capsule postérieure. Dans ce cas, une nouvelle intervention, toutefois non invasive, est nécessaire pour ouvrir la partie centrale de la capsule postérieure, notamment par laser YAG pour écarter la zone centrale de la capsule postérieure.

L'élimination des cellules épithéliales lors de l'extraction du cristallin est un problème qui n'a pas été résolu de manière satisfaisante.

On connaît par le document EP 283 929 un instrument à main pour le nettoyage du sac capsulaire après extraction du cristallin comportant un dispositif de d'irrigation du sac capsulaire et un dispositif d'aspiration des débris entraînés par le fluide. Il comporte un premier tube creux qui délimite un canal d'aspiration terminé par un bout arrondi et un deuxième tube entourant le premier tube de manière coaxiale, terminé par un rétrécissement joint au premier tube avant l'extrémité au bout arrondi du premier tube. Le premier tube est connecté à une pompe d'aspiration. Un canal d'irrigation est créé entre les deux tubes. Le premier tube comporte un orifice d'aspiration pratiqué sur la paroi cylindrique à proximité du bout arrondi. Le deuxième tube comporte deux orifices d'irrigation pratiqués sur la paroi cylindrique à proximité du rétrécissement. Ces deux orifices d'irrigation sont disposés de manière symétrique par rapport à un plan axial de l'instrument contenant l'axe de l'orifice d'aspiration. L'instrument comporte en outre un dépôt abrasif sur la surface extérieure du premier tube, à l'opposé de l'orifice d'aspiration qui sert à gratter les cellules épithéliales de la surface intérieure du sac capsulaire.

Cet instrument est utilisé après l'extraction du cristallin, notamment par phaco-émulsification, pour éliminer les résidus contenus dans le sac capsulaire. Pour cela, l'extrémité de l'instrument est introduite dans le sac en passant d'abord par une incision cornéenne et ensuite l'ouverture centrale pratiquée dans la capsule antérieure appelée rhexis. Un liquide est amené par le canal d'irrigation dans le sac capsulaire à travers les deux orifices d'irrigation. Ce liquide est aspiré à travers l'orifice d'aspiration et le canal d'aspiration. Les résidus entraînés par ce liquide sont évacués du sac capsulaire. Le chirurgien gratte les surfaces intérieures des capsules du sac capsulaire avec la surface dotée d'une couche abrasive afin d'essayer de "décoller" les cellules épithéliales.

Cette opération délicate est conduite sous observation par microscope, au travers de l'espace visuel laisser libre par la pupille. En outre, l'iris masque la zone équatoriale du sac capsulaire. L'accès à cette zone équatoriale masquée est difficile et présente des risques pour l'intégrité du sac capsulaire. Des cellules épithéliales subsistent donc à l'intérieur du sac capsulaire, et les risques de cataracte secondaire ne sont pas éliminés, du fait de la migration des cellules et de leur métaplasie fibroblastique, restent un problème majeur de la chirurgie de la cataracte.

Par ailleurs, le rayon d'action de l'instrument étant relativement faible, l'opérateur est conduit à placer l'orifice d'aspiration très près des capsules à gratter ou nettoyer. Ceci crée un risque d'aspiration de la capsule concernée pouvant conduire à sa perforation ou à son déchirement.

Par ailleurs le document US 5 024 654 décrit un instrument de chirurgie pour le nettoyage du sac capsulaire après extraction du cristallin selon le préambule de la revendication 1.

C'est donc un objet de l'invention de fournir un instrument permettant une meilleure élimination des cellules épithéliales, y compris dans la zone équatoriale du sac capsulaire.

A cet fin, il est pourvu un instrument de chirurgie pour le nettoyage du sac capsulaire après extraction du cristallin, selon la revendication 1.

Grâce à cet instrument, on peut créer un jet de liquide dans le sac capsulaire et l'orienter contre une surface interne du sac capsulaire. Ce jet permet de décoller et emporter les cellules épithéliales accrochées aux surfaces internes du sac capsulaire. De plus, comme le jet est dirigé vers l'avant du premier tube, son rayon d'action dépasse son extrémité avant. Ainsi, il est possible d'enlever les cellules épithéliales et nettoyer le sac capsulaire vers l'avant et sur les surfaces du sac disposées latéralement par rapport à l'axe du premier tube et dans la zone équatoriale, éventuellement avec l'extrémité de l'instrument restant dans une zone visible à travers la pupille. En tout cas, l'intérieur du sac capsulaire peut donc être nettoyé de manière efficace pour enlever un maximum des cellules épithéliales.

Selon un second aspect de l'invention, il est pourvu un instrument de chirurgie ophtalmique, comportant un premier tube d'alimentation de liquide ayant au moins un orifice de jet et un moyen d'amenée du liquide sous pression au premier tube d'alimentation, caractérisé en ce que la pression du liquide à l'orifice de jet est entre environ 20 000 Pa et 70 000 Pa.

De préférence, l'axe de l'orifice de jet est incliné d'un angle de jet compris entre 20° et 70°, et plus particulièrement de 45°, par rapport à l'axe du premier tube et dans un plan longitudinal.

Selon l'invention, le diamètre de l'orifice de jet est compris entre 50 et 150 µm, et de préférence de 100 µm. Ainsi, le jet généré est suffisamment large pour contrer les effets de la viscosité du liquide et les forces capillaires et avoir une zone d'action suffisamment importante tout en restant suffisamment étroite pour s'insinuer entre les amas cellulaires pour les détacher. Par ailleurs, le débit de liquide passant par cet orifice peut rester relativement faible et n'a pas d'influence notable sur la pression à l'intérieur du sac capsulaire pendant l'intervention.

De préférence, le diamètre intérieur du tube d'alimentation est compris entre 800 et 1200 µm. Ainsi, le tube est suffisamment grand pour que les pertes de charge de l'écoulement d'un liquide soient faibles, mais suffisamment petit pour que l'extrémité de l'instrument soit introduite dans le sac capsulaire à travers une incision cornéenne de faible taille. En comparant la section de passage du liquide dans le tube d'alimentation et celle de l'orifice, on constate un rapport de 20 à plus de 24. Ainsi, les pertes de charge dans le tube d'alimentation sont faibles et on retrouve pratiquement la même pression à la source du liquide et en amont de l'orifice. De même, l'épaisseur de la paroi du premier tube entourant l'(ou les) orifice(s) est réduite pour diminuer la perte de charge au niveau de l'orifice. A cette fin, l'épaisseur est de préférence égale ou inférieure au diamètre de l'orifice de jet ou entre environ 50 et 150 µm.

Selon un mode de réalisation particulier, le tube d'alimentation est coudé à proximité de son extrémité avant. Ainsi, il est possible d'accéder plus facilement à la surface du sac capsulaire au voisinage du rhexis dans la capsule antérieure par lequel l'extrémité de l'instrument est introduite.

Selon un mode de réalisation particulier, l'instrument incorpore en outre un tube d'aspiration coaxial au tube d'alimentation, le tube d'aspiration comportant à son extrémité avant un rétrécissement joint au tube d'alimentation en saillie de l'extrémité avant du tube d'alimentation.

De préférence, l'instrument est réalisé en matériau choisi parmi un matériau organique polymère, un acier inoxydable, du titane ou du tungstène.

De manière particulière, l'instrument comporte des moyens d'obturation du tube d'alimentation. Ainsi, le chirurgien pourra commander l'arrivée du liquide à l'orifice de jet. En particulier, l'arrivée de liquide sera interrompue lors de l'introduction de l'instrument dans l'oeil et ensuite dans le sac capsulaire, pour éviter d'endommager les tissus oculaires, notamment à proximité du rhexis.

De manière avantageuse, l'instrument comporte des moyens de connexion d'arrivée pour relier le tube d'alimentation à des moyens d'amenée du liquide. Ces moyens de connexion sont par exemple des connecteurs standard de raccordement à un réservoir tels qu'un dispositif pour perfusion. Tout autre moyen de connexion pour contrôler l'amenée de liquide peut être adopté.

De manière particulière, le tube d'alimentation comporte des repères visuels externes répartis le long de l'axe du tube d'alimentation. Ainsi, l'opérateur peut estimer la position de l'extrémité masquée de l'instrument en identifiant le repère le plus proche du rhexis, par exemple.

L'invention sera mieux comprise et d'autres particularités et avantages apparaîtront à la lecture de la description qui va suivre, la description faisant référence aux dessins annexés parmi lesquels :
- la figure 1 est une vue d'un instrument mettant en oeuvre différents aspects de l'invention ;
- la figure 2 est une vue en coupe du détail II de la figure 1 ;
- les figures 3, 4 et 5 sont des vues de bout de l'instrument de la figure 1 selon trois variantes ;
- la figure 6 est une vue similaire à la figure 2 d'un deuxième mode de réalisation de l'invention
- la figure 7 est une vue de l'extrémité d'un instrument selon un troisième mode de réalisation de l'invention.

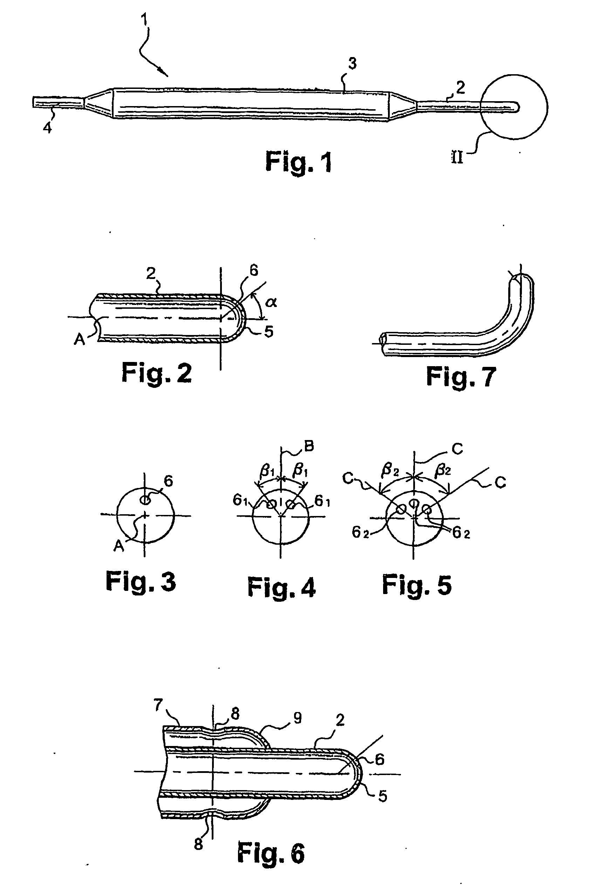

L'instrument 1 des figures 1 à 3 comporte un tube d'alimentation droit 2 porté par une poignée 3 et prolongé à l'arrière par un embout 4. Le tube d'alimentation 2 est fermé à une extrémité avant par un bout 5, de préférence le bout avant. Le tube d'alimentation 2 est destiné à être introduit dans le sac capsulaire de l'oeil après l'extraction du cristallin. La paroi du tube d'alimentation a une épaisseur de l'ordre de 100 µm. L'embout 4 est destiné à recevoir un conduit amenant un liquide à l'instrument. Un canal d'alimentation s'étend depuis l'embout 4 jusqu'à l'extrémité avant à l'intérieur du tube d'alimentation 2. Comme on peut le voir en particulier sur les figures 2 et 3, le bout avant 5 du tube d'alimentation 2 comporte un orifice 6. Dans un plan longitudinal tel que le plan de coupe de la figure 2, l'axe de l'orifice 6 forme un angle de jet α avec l'axe A de l'instrument. L'angle de jet α a une valeur de l'ordre de 20 à 70° et de préférence 45°, de telle sorte que l'orifice 6 débouche latéralement ou radialement et vers l'avant du tube d'alimentation 2.

Dans une variante de l'instrument 1, qui est montrée sur la figure 4, le bout arrondi 5 comporte deux orifices 6₁ placés de manière symétrique par rapport à un plan longitudinal B de l'instrument 1, et selon un angle β₁ entre les plans longitudinaux contenant les axes des orifices 6₁ et le plan B. L'angle β₁ vaut par exemple 15°.

Dans une autre variante représentée sur la figure 5, le bout avant 5 comporte trois orifices 6₂. Les axes des orifices sont placés respectivement dans trois plans longitudinaux C, décalés entre eux d'un angle β₂. L'angle β₂ est d'environ par exemple de 30 °.

Dans un deuxième mode de réalisation, représenté sur la figure 6, l'instrument incorpore également un dispositif d'aspiration. A cet effet, le tube d'alimentation 2 est entouré d'un tube d'aspiration 7, et qui se termine par un rétrécissement 9. Le rétrécissement 9 rejoint le tube d'alimentation 2 à proximité de l'extrémité avant du tube d'alimentation 2. Le tube d'aspiration 7 comporte au moins un orifice d'aspiration 8 situé sur la partie cylindrique du tube d'aspiration 7 et à proximité du rétrécissement 9.

Dans un troisième mode de réalisation, montré sur la figure 7, l'extrémité de l'instrument incorporant ou non un dispositif d'aspiration présente un coude à 90°. L'axe de l'orifice est situé dans le même plan que le coude. Cependant, des variantes peuvent être envisagées dans lesquelles le coude est moins prononcé, par exemple 45°, ou dans lesquelles l'axe de l'orifice n'est pas dans le plan du coude.

Cet instrument 1 est utilisé lors des opérations de la cataracte après la phase d'extraction du cristallin, pour éliminer le maximum de cellules épithéliales contenues dans le sac capsulaire. Lors de l'utilisation, l'embout 4 est connecté à un conduit qui amène à l'instrument 1 un liquide qui peut être une solution saline équilibrée. Le liquide est par exemple contenu dans un réservoir du type dispositif de perfusion placé en hauteur par rapport au champ opératoire. Dans ce cas, la pression d'arrivée du liquide au niveau de l'orifice de jet sera égale ou supérieure à environ 20 000 Pa et inférieure à environ 70 000 Pa. En effet, une pression supérieure à 70 000 Pa risquerait d'endommager le sac capsulaire. Toutefois, de préférence, l'alimentation du liquide est assurée par une pompe ou une seringue électrique dont le débit est calibré en fonction de l'instrument pour obtenir la pression souhaitée au niveau de l'orifice de jet. Dans ce cas, la pression sera comprise entre environ 20 000 Pa et environ 70 000 Pa, de préférence entre environ 30 000 Pa et 50 000 Pa, et plus préférentiellement d'environ 35 000 Pa et 45 000 Pa et en pratique de l'ordre de 40 000 Pa.

Une fois que le cristallin a été émulsifié et évacué à travers le rhexis et l'incision cornéenne, l'extrémité avant de l'instrument est introduite dans le sac capsulaire pour son nettoyage. Le liquide est amené et sort de l'extrémité avant de l'instrument par l'orifice 6. Ainsi, un jet est formé et orienté latéralement ou radialement et vers l'avant de l'extrémité avant de l'instrument. Les cellules épithéliales encore accrochées au sac capsulaire sont soumises au jet et détachées sous l'effet de la force exercée par le jet de liquide.

Le liquide amené par l'instrument peut être évacué du sac à travers le rhexis pratiqué dans la capsule antérieure et par laquelle passe l'instrument 1. On peut utiliser une sonde d'aspiration distincte de l'instrument qui est introduite également dans le sac capsulaire, par le rhexis ou éventuellement par une autre incision dans le sac capsulaire, et qui aspire le liquide amené dans le sac capsulaire par l'instrument. Le liquide évacué entraîne avec lui les cellules détachées.

Dans le deuxième mode de réalisation de l'invention, l'instrument de la figure 6 incorpore un dispositif d'aspiration comportant en plus un tube d'aspiration 7, en plus du dispositif d'alimentation. Dans ce cas, le liquide amené par le conduit d'alimentation et qui sort par l'orifice 6 est aspiré par l'orifice d'aspiration 8 pour être évacué, grâce à une pompe à vide, non représentée, connectée au conduit d'aspiration.

Dans les variantes des figures 4 et 5, l'instrument comporte plusieurs orifices de jet. Ceux-ci permettent d'agir sur une zone plus importante et réduire le temps de l'opération de nettoyage du sac capsulaire.

Dans le mode de réalisation de la figure 7, la partie coudée permet d'accéder à des zones du sac capsulaire de part et d'autre de l'incision par laquelle l'instrument est introduit.

Selon une autre variante, l'extrémité avant de l'instrument peut comporter une zone dépolie sur une partie cylindrique du tube d'alimentation 2 ainsi que sur une partie du bout arrondi 5. Cette zone dépolie permet d'effectuer un grattage local complémentaire pour détacher les cellules encore accrochées au sac capsulaire. On remarque que la zone de grattage est relativement éloignée de l'orifice d'aspiration, empêchant ainsi, en pratique, la perforation ou le déchirement par l'action d'aspiration.

Selon une autre variante, le tube d'alimentation 2 comporte sur sa surface extérieure des marquages en forme d'anneau, qui graduent l'extrémité avant du tube, par exemple en millimètres. Ces anneaux peuvent avoir des couleurs différentes ou un marquage différencié permettant de déterminer 'évaluer la distance entre l'anneau et l'extrémité avant du tube. Ainsi, l'opérateur peut connaître la longueur de l'instrument qui a pénétré dans le sac capsulaire sans contrôle visuel, c'est-à-dire masqué par l'iris.

Il va de soi que la présente invention n'est pas limitée aux formes de réalisation décrites ni aux matériaux préférés, mais englobe au contraire toutes variantes de structures et configurations et de matériaux compatibles avec les objets de la présente invention.

## Revendications

1. Instrument de chirurgie ophtalmique pour le nettoyage du sac capsulaire après extraction du cristallin, comportant un premier tube (2) d'alimentation, l'instrument étant configuré pour permettre son introduction dans le sac capsulaire, **caractérisé en ce que** le tube d'alimentation est fermé à une extrémité avant par un bout avant, le bout avant (5) du premier tube a au moins un orifice de jet (6) débouchant à la fois latéralement et vers l'avant du premier tube pour créer un jet de liquide permettant de décoller les cellules épithéliales accrochées aux surfaces internes du sac capsulaire et **caractérisé en ce que** le diamètre de l'orifice de jet est compris entre 50 et 150 µm.

2. Instrument de chirurgie ophtalmique selon la revendication 1, **caractérisé en ce que** l'axe de l'orifice de jet (6) est incliné d'un angle de jet (α) compris entre 20° et 70°, par rapport à l'axe du premier tube (2) et dans son plan longitudinal.

3. Instrument de chirurgie ophtalmique selon la revendication 1, **caractérisé en ce que** l'angle de jet (α) est de 45°.

4. Instrument de chirurgie ophtalmique selon l'une quelconque des revendications 1 à 3, **caractérisé en ce que** le diamètre de l'orifice de jet est sensiblement de 100 µm.

5. Instrument de chirurgie ophtalmique selon l'une quelconque des revendications 1 à 4, **caractérisé en ce que** l'épaisseur de la paroi du tube d'alimentation entourant l'orifice de jet (6) est égale ou inférieure au diamètre de l'orifice de jet.

6. Instrument de chirurgie ophtalmique selon l'une quelconque des revendications 1 à 4, **caractérisé en ce que** l'épaisseur de la paroi du tube d'alimentation entourant l'orifice de jet (6) est entre environ 50 et 150 µm.

7. Instrument de chirurgie ophtalmique selon l'une quelconque des revendications 1 à 6, **caractérisé en ce que** le diamètre intérieur du tube d'alimentation est compris entre 800 et 1200 µm.

8. Instrument de chirurgie ophtalmique selon l'une quelconque des revendications précédentes, **caractérisé en ce que** le tube d'alimentation est coudé à proximité de son extrémité avant.

9. Instrument de chirurgie ophtalmique selon l'une quelconque des revendications précédentes, **caractérisé en ce qu'**il incorpore un tube d'aspiration (7) coaxial au tube d'alimentation (2), le tube d'aspiration (7) comportant à son extrémité un rétrécissement (9) joint au tube d'alimentation (2) avant l'extrémité avant du tube d'alimentation (2).

10. Instrument de chirurgie ophtalmique selon l'une quelconque des revendications précédentes, **caractérisé en ce qu'**il est réalisé en matériau choisi parmi un matériau organique polymère, un acier inoxydable, du titane ou du tungstène.

11. Instrument de chirurgie ophtalmique selon l'une quelconque des revendications précédentes, **caractérisé en ce qu'**il comporte des moyens d'obturation du tube d'alimentation (2).

12. Instrument de chirurgie ophtalmique selon l'une quelconque des revendications précédentes, **caractérisé en ce qu'**il comporte des moyens de connexion d'arrivée (4) pour relier le tube d'alimentation (2) à des moyens d'amenée d'un liquide.

13. Instrument de chirurgie ophtalmique selon l'une quelconque des revendications précédentes, **caractérisé en ce que** le tube d'alimentation (2) comporte des repères visuels externes répartis le long de l'axe A du tube d'alimentation (2).

14. Instrument de chirurgie ophtalmique selon l'une quelconque des revendications 1 à 15, **caractérisé en ce que** la pression du liquide à la sortie de l'orifice de jet (6) est entre environ 20 000 Pa et 70 000 Pa.

15. Instrument de chirurgie ophtalmique selon l'une quelconque des revendications 1 à 13, **caractérisé en ce que** la pression du liquide à la sortie de l'orifice de jet (6) est entre environ 30 000 Pa et 50 000 Pa.

16. Instrument de chirurgie ophtalmique selon l'une quelconque des revendications 1 à 13, **caractérisé en ce que** la pression du liquide à la sortie de l'orifice de jet (6) est entre 35 000 Pa et 45 000 Pa.

17. Instrument de chirurgie ophtalmique selon l'une quelconque des revendications 1 à 13, **caractérisé en ce que** la pression du liquide à la sortie de l'orifice de jet (6) est de 40 000 Pa.

18. Instrument de chirurgie ophtalmique selon l'une quelconque des revendications 1 à 17, la paroi intérieure du tube d'alimentation définissant la section d'écoulement du liquide dans ledit tube.

19. Instrument de chirurgie ophtalmique selon l'une quelconque des revendications 1 à 18, **caractérisé en ce que** le bout avant du tube d'alimentation est arrondi.

20. Instrument de chirurgie ophtalmique selon l'une quelconque des revendications 1 à 18, **caractérisé en ce que** l'extrémité avant de l'instrument comporte une zone dépolie sur une partie cylindrique du tube d'alimentation et sur le bout avant.

21. Instrument de chirurgie ophtalmique selon l'une quelconque des revendications 1 à 18, **caractérisé en ce que** le rapport entre la section de passage du liquide dans le tube d'alimentation et celle de l'orifice est de 20 à plus de 24.

## Claims

1. Ophthalmic surgical instrument for cleaning the capsular bag after extraction of the crystalline lens, including a first irrigation tube (2), the instrument being configured to enable its insertion into the capsular bag, **characterised in that** the irrigation tube is closed at a front end by a front tip, the front tip (5) of the first tube has at least one jet orifice (6) discharging both laterally and towards the front of the first tube to create a jet of liquid enabling detachment of the epithelial cells attached to the internal surfaces of the capsular bag and **characterised in that** the diameter of the jet orifice is in the range between 50 and 150 µm.

2. Ophthalmic surgical instrument according to claim 1, **characterised in that** the axis of the jet orifice (6) is inclined at a jet angle (α) in the range between 20° and 70° relative to the axis of the first tube (2) and in its longitudinal plane.

3. Ophthalmic surgical instrument according to claim 1, **characterised in that** the jet angle (α) is 45°.

4. Ophthalmic surgical instrument according to any one of claims 1 to 3, **characterised in that** the diameter of the jet orifice is substantially equal to 100 µm.

5. Ophthalmic surgical instrument according to any one of claims 1 to 4, **characterised in that** the thickness of the wall of the irrigation tube surrounding the jet orifice (6) is equal to or less than the diameter of the jet orifice.

6. Ophthalmic surgical instrument according to any one of claims 1 to 4, **characterised in that** the thickness of the wall of the irrigation tube surrounding the jet orifice (6) is in the range approximately 50 to 150 µm.

7. Ophthalmic surgical instrument according to any one of claims 1 to 6, **characterised in that** the inside diameter of the irrigation tube is in the range between 800 and 1200 µm.

8. Ophthalmic surgical instrument according to any one of the preceding claims, **characterised in that** the irrigation tube is elbowed in the vicinity of its front end.

9. Ophthalmic surgical instrument according to any one of the preceding claims, **characterised in that** it incorporates an aspiration tube (7) coaxial with the irrigation tube (2), the aspiration tube (7) having at its end a portion (9) reduced in size joined to the irrigation tube (2) in front of the front end of the irrigation tube (2).

10. Ophthalmic surgical instrument according to any one of the preceding claims, **characterised in that** it is produced in a material chosen from an organic polymer material, stainless steel, titanium or tungsten.

11. Ophthalmic surgical instrument according to any one of the preceding claims, **characterised in that** it includes means for closing of the irrigation tube (2).

12. Ophthalmic surgical instrument according to any one of the preceding claims, **characterised in that** it includes inlet connecting means (4) for connecting the irrigation tube (2) to fluid supply means.

13. Ophthalmic surgical instrument according to any one of the preceding claims, **characterised in that** the irrigation tube (2) includes external visual markers distributed along the axis A of the irrigation tube (2).

14. Ophthalmic surgical instrument according to any one of claims 1 to 15, **characterised in that** the pressure of the liquid at the outlet of the jet orifice (6) is in the range approximately between 20 000 Pa and 70 000 Pa.

15. Ophthalmic surgical instrument according to any one of claims 1 to 13, **characterised in that** the pressure of the liquid at the outlet of the jet orifice (6) is in the range approximately between 30 000 Pa and 50 000 Pa.

16. Ophthalmic surgical instrument according to any one of claims 1 to 13, **characterised in that** the pressure of the liquid at the outlet of the jet orifice (6) is in the range between 35 000 Pa and 45 000 Pa.

17. Ophthalmic surgical instrument according to any one of claims 1 to 13, **characterised in that** the pressure of the liquid at the outlet of the jet orifice (6) is 40 000 Pa.

18. Ophthalmic surgical instrument according to any one of claims 1 to 17, the interior wall of the irrigation tube defining the flow section of the liquid in said tube.

19. Ophthalmic surgical instrument according to any one of claims 1 to 18, **characterised in that** the front tip of the irrigation tube is rounded.

20. Ophthalmic surgical instrument according to any one of claims 1 to 18, **characterised in that** the front end of the instrument includes an unpolished area on a cylindrical portion of the irrigation tube and on the front tip.

21. Ophthalmic surgical instrument according to any one of claims 1 to 18, **characterised in that** the ratio between the flow section of the liquid in the irrigation tube and that of the orifice is in the range of 20 to more than 24.

## Patentansprüche

1. Chirurgisches Ophthalmieinstrument zur Reinigung des Kapselsacks nach der Extraktion der Linse, das ein erstes Versorgungsrohr (2) umfasst, wobei das Instrument konfiguriert ist, um seine Einführung in den Kapselsack zu ermöglichen, **dadurch gekennzeichnet, dass** das Versorgungsrohr an einem vorderen Ende durch einen vorderen Stopfen verschlossen ist, wobei das vordere Ende (5) des ersten Rohrs wenigstens eine Strahlmündung (6) besitzt, die sowohl seitlich als auch zur Vorderseite des ersten Rohrs mündet, um einen Flüssigkeitsstrahl zu erzeugen, der ermöglicht, die Epithelzellen zu lösen, die an den inneren Oberflächen des Kapselsacks anhaften, und dadurch, dass der Durchmesser der Strahlmündung im Bereich von 50 bis 150 µm liegt.

2. Chirurgisches Ophthalmieinstrument nach Anspruch 1, **dadurch gekennzeichnet, dass** die Achse der Strahlmündung (6) unter einem Strahlwinkel (α) im Bereich von 20° bis 70° in Bezug auf die Achse des ersten Rohrs (2) und in dessen longitudinaler Ebene geneigt ist.

3. Chirurgisches Ophthalmieinstrument nach Anspruch 1, **dadurch gekennzeichnet, dass** der Strahlwinkel (α) 45° beträgt.

4. Chirurgisches Ophthalmieinstrument nach einem der Ansprüche 1 bis 3, **dadurch gekennzeichnet, dass** der Durchmesser der Strahlmündung im Wesentlichen 100 µm beträgt.

5. Chirurgisches Ophthalmieinstrument nach einem der Ansprüche 1 bis 4, **dadurch gekennzeichnet, dass** die Dicke der Wand des Versorgungsrohrs, die die Strahlmündung (6) umgibt, gleich oder kleiner dem Durchmesser der Strahlmündung ist.

6. Chirurgisches Ophthalmieinstrument nach einem der Ansprüche 1 bis 4, **dadurch gekennzeichnet, dass** die Dicke der Wand des Versorgungsrohrs, die die Strahlmündung (6) umgibt, im Bereich von etwa 50 bis 150 µm liegt.

7. Chirurgisches Ophthalmieinstrument nach einem der Ansprüche 1 bis 6, **dadurch gekennzeichnet, dass** der Innendurchmesser des Versorgungsrohrs im Bereich von 800 bis 1200 µm liegt.

8. Chirurgisches Ophthalmieinstrument nach einem der vorhergehenden Ansprüche, **dadurch gekennzeichnet, dass** das Versorgungsrohr in der Umgebung seines vorderen Endes gebogen ist.

9. Chirurgisches Ophthalmieinstrument nach einem der vorhergehenden Ansprüche, **dadurch gekennzeichnet, dass** es ein Saugrohr (7) koaxial zu dem Versorgungsrohr (7) umfasst, wobei das Saugrohr (7) an seinem Ende eine Einschnürung (9) aufweist, die mit dem Versorgungsrohr (2) vor dem vorderen Ende des Versorgungsrohrs (2) verbunden ist.

10. Chirurgisches Ophthalmieinstrument nach einem der vorhergehenden Ansprüche, **dadurch gekennzeichnet, dass** es aus einem Material hergestellt ist, das aus einem organischen Polymermaterial, einem rostfreien Stahl, Titan oder Wolfram gewählt ist.

11. Chirurgische Ophthalmieinstrument nach einem der vorhergehenden Ansprüche, **dadurch gekennzeichnet, dass** es Mittel zum Verschließen des Versorgungsrohrs (2) umfasst.

12. Chirurgisches Ophthalmieinstrument nach einem der vorhergehenden Ansprüche, **dadurch gekennzeichnet, dass** es Einlassverbindungsmittel (4) umfasst, um das Versorgungsrohr (2) mit Flüssigkeitszufuhrmitteln zu verbinden.

13. Chirurgisches Ophthalmieinstrument nach einem der vorhergehenden Ansprüche, **dadurch gekennzeichnet, dass** das Versorgungsrohr (2) äußere visuelle Markierungen aufweist, die längs der Achse A des Versorgungsrohrs (2) verteilt sind.

14. Chirurgisches Ophthalmieinstrument nach einem der Ansprüche 1 bis 15, **dadurch gekennzeichnet, dass** der Druck der Flüssigkeit am Ausgang der Strahlmündung (6) im Bereich von etwa 20000 Pa bis 70000 Pa liegt.

15. Chirurgisches Ophthalmieinstrument nach einem der Ansprüche 1 bis 13, **dadurch gekennzeichnet, dass** der Druck der Flüssigkeit am Ausgang der Strahlmündung (6) im Bereich von etwa 30000 Pa bis 50000 Pa liegt.

16. Chirurgisches Ophthalmieinstrument nach einem der Ansprüche 1 bis 13, **dadurch gekennzeichnet, dass** der Druck der Flüssigkeit am Ausgang der Strahlmündung (6) im Bereich von 35000 Pa bis 45000 Pa liegt.

17. Chirurgisches Ophthalmieinstrument nach einem der Ansprüche 1 bis 13, **dadurch gekennzeichnet, dass** der Druck der Flüssigkeit am Ausgang der Strahlmündung (6) 40000 Pa beträgt.

18. Chirurgisches Ophthalmieinstrument nach einem der Ansprüche 1 bis 17, wobei die Innenwand des Versorgungsrohrs den Strömungsquerschnitt der Flüssigkeit in dem Rohr definiert.

19. Chirurgisches Ophthalmieinstrument nach einem der Ansprüche 1 bis 18, **dadurch gekennzeichnet, dass** das vordere Ende des Versorgungsrohrs abgerundet ist.

20. Chirurgisches Ophthalmieinstrument nach einem der Ansprüche 1 bis 18, **dadurch gekennzeichnet, dass** das vordere Ende des Instruments eine matt geschliffene Zone auf einem zylindrischen Teil des Versorgungsrohrs und auf dem vorderen Ende aufweist.

21. Chirurgisches Ophthalmieinstrument nach einem der Ansprüche 1 bis 18, **dadurch gekennzeichnet, dass** das Verhältnis zwischen dem Flüssigkeitsdurchlassquerschnitt in dem Versorgungsrohr und jenem der Mündung im Bereich von 20 bis mehr als 24 liegt.
